# EUROPEAN PATENT APPLICATION

(11) **EP 4 166 498 A1**
(43) Date of publication of application: **19.04.2023**
(21) Application number: 21913256.0
(22) Date of filing: 23.09.2021
(51) Int. Cl.: B81C 1/00, B29C 33/38, B29C 39/02

(54) **MANUFACTURING METHOD FOR 3D MICROELECTRODE**

(30) Priority: 30.12.2020 CN 202011622362
(71) Applicant: Harbin Institute of Technology, Shenzhen, Shenzhen, Guangdong 518055 (CN)
(72) Inventor: ZHU, Yonggang, Shenzhen, Guangdong 518055 (CN); CHEN, Chaozhan, Shenzhen, Guangdong 518055 (CN); CHEN, Huaying, Shenzhen, Guangdong 518055 (CN); RAN, Bin, Shenzhen, Guangdong 518055 (CN); LIU, Bo, Shenzhen, Guangdong 518055 (CN); LV, Chuanwen, Shenzhen, Guangdong 518055 (CN)
(74) Representative: Botti, Mario
(86) International application number: PCT/CN2021/119872
(87) International publication number: WO 2022/142511

(57) **Abstract**

Disclosed in the present disclosure is a manufacturing method for a 3D microelectrode. The manufacturing method comprises the following steps: (1) manufacturing a 3D model of a 3D microelectrode; (2) pouring a flexible material into the 3D model, and performing demolding so as to form a flexible mold having a cavity, wherein the cavity of the flexible mold can be fitted to the 3D model; (3) performing silanization treatment on the flexible mold, then pouring a flexible material into the surface of the flexible mold having the cavity, and performing demolding so as to form a flexible 3D microelectrode substrate; and (4) manufacturing a conductive layer on the flexible 3D microelectrode substrate so as to form the 3D microelectrode. In the present disclosure, a 3D microelectrode having an ultrahigh microcolumn height can be manufactured by using a 3D printing technology and a two-time mold-reversing method; furthermore, a flexible material is used as a substrate, so that the formed 3D microelectrode has the characteristics of low costs, rapidness, high precision and flexibility, and can be used in the field of electrochemical analysis on wearable devices.

## Description

### CROSS-REFERENCE TO RELATED APPLICATION

This patent application claims the priority of Chinese Patent Application CN202011622362.6 filed with the China National Intellectual Property Administration on December 30, 2020, and entitled "Manufacturing method for 3D Microelectrode", the disclosure of which are entirely incorporated by reference herein.

### TECHNICAL FIELD

The present disclosure relates to the preparation of electrodes, and in particular relates to a manufacturing method for a 3D microelectrode.

### BACKGROUND

Electrochemical study is an analysis method for characterization and measurement by using the electrochemical properties of substances. By employing the electrochemical analysis method, not only can the analysis result be automatically recorded, but also the detection of trace substances, including glucose, sarcosine, urea and the like, is facilitated, and such method is widely applied to the aspects of industry, agriculture, food safety and the like.

At present, the microcolumn electrode array is mainly fabricated by using a complex photolithography technology, including LIGA-like processes, carbonization of photoresist patterned on a substrate, and homoepitaxial growth. Prehn et al., have reported the fabrication of microcolumn electrode array having a column height of 10 µm by using photolithography, metallization and electrodeposition technologies. Secondly, Sanchez-Molas et al., manufactured microcolumn electrode array with higher microcolumn (maximum 125 µm) with sputtering and deep reactive ion etching (DRIE), which showed better clarity and repeatability. However, the fabrication process is not only expensive but also time-consuming. In addition, both aspect ratio and column height are limited by lithographic processes. Moreover, the electrochemical detection sensor is simply manufactured by using 3D technique, expensive 3D printer needs to be purchased, and the time consumed for printing the sensor is long. The manufacturing of microcolumn electrode array with higher microcolumns is essential for the development of low-cost and high-sensitive microsensors for chemical and biological substances as the larger electrode surface area can be obtained by manufacturing high microcolumn heights and the large electrode surface area is conducive to acquiring larger response current.

### SUMMARY

The present disclosure aims at solving at least one of the technical problems existing in the prior art. To this end, the present disclosure provides a manufacturing method for a 3D microelectrode, which can be used for manufacturing an ultrahigh microcolumn electrode array as high as 500 µm to 2 mm, has the characteristics of low costs, rapidness, high precision and flexibility, and can be used in the field of electrochemical analysis on wearable devices.

To achieve the objective above of the present disclosure, the present disclosure provides the following technical solution:

In a first aspect of the present disclosure, a manufacturing method for a 3D microelectrode is provided, comprising the following steps:
(1) manufacturing a 3D model of a 3D microelectrode;
(2) pouring a flexible material into the 3D model, and performing demolding so as to form a flexible mold having a cavity, wherein the cavity of the flexible mold can be fitted to the 3D model;
(3) performing silanization treatment on the flexible mold, then pouring a flexible material into the surface of the flexible mold having the cavity, and performing demolding so as to form a flexible 3D microelectrode substrate; and
(4) manufacturing a conductive layer on the flexible 3D microelectrode substrate so as to form the 3D microelectrode.

Preferably, the flexible material is selected from any one of PDMS (Polydimethylsiloxane), PET (polyethylene terephthalate) and polyimide.

Preferably, the flexible material is a PDMS solution, and a mass ratio of a PDMS prepolymer to a curing agent in the PDMS solution is 10: 1.

Preferably, the 3D microelectrode is an electrode array.

Preferably, a single electrode in the electrode array is a circular truncated-cone-shaped electrode, a conical electrode, a cylindrical electrode, a triangular prism-shaped electrode, a prism-shaped electrode, or a spherical electrode.

Preferably, the circular truncated-cone-shaped electrodes each have a bottom circle radius of 10 µm to 100 µm and a height of 100 µm to 2 mm, and the distance between the truncated-cone-shaped electrodes is 100 µm to 500 µm.

Preferably, the column height of a single electrode in the electrode array ranges from 5 µm to 2 mm.

Preferably, the 3D model of the 3D microelectrode is manufactured by using a 3D printing technology.

Preferably, the conductive layer in the step (4) is a conductive metal layer or a conductive polymer layer.

Preferably, the conductive layer has a thickness of 150 nm to 250 nm.

Preferably, the conductive metal layer is made of gold, platinum or indium tin oxide.

Preferably, in the step (4), the conductive metal layer is manufactured by using a magnetron sputtering process, or the conductive polymer layer is manufactured by coating conductive polymer.

Preferably, the 3D microelectrode is provided with a substrate portion and a protruded portion fixed on the substrate portion, and the method further comprises a step of manufacturing a non-conductive isolation layer on the substrate portion.

Preferably, the non-conductive isolation layer is made of at least one of silicon nitride, silicon dioxide and a non-conductive polymer.

Preferably, the non-conductive isolation layer is manufactured on the substrate portion by using chemical vapor deposition and lift-off technologies.

In accordance with the manufacturing method for the 3D microelectrode of the embodiment of the present disclosure, at least the following beneficial effects are obtained: According to the embodiment of the present disclosure, the morphology of a 3D microelectrode is copied and obtained by pouring a flexible material for the first time, then silanization treatment is performed to form a layer of polymer film on the surface of a flexible mold. After the flexible material is poured for the second time, two-time model separation can be achieved through the polymer film. A 3D microelectrode having an ultrahigh microcolumn height can be manufactured by a two-time mold-reversing method; furthermore, a flexible material is used as a substrate, so that the formed 3D microelectrode has the characteristics of low costs, rapidness, high precision and flexibility, and can be used in the field of electrochemical analysis on wearable devices.

### BRIEF DESCRIPTION OF THE DRAWINGS

FIG. 1 is a schematic diagram of a structure of a 3D microelectrode in accordance with an embodiment of the present disclosure;
FIG. 2 is a partial enlarged view of a portion A in FIG. 1;
FIG. 3 is a schematic diagram of a manufacturing process of a 3D microelectrode in accordance with the embodiment of the present disclosure;
FIG. 4 is a schematic diagram of a structure of a 3D microelectrode with a non-conductive isolation layer in accordance with the embodiment of the present disclosure;

In the drawings: 100-3D model; 200-PDMS flexible mold; 210-cavity; 300-PDMS flexible 3D microelectrode substrate; 400-conductive layer; 510-protruded portion; 520-substrate portion; 600-non-conductive isolation layer.

### DETAILED DESCRIPTION OF THE EMBODIMENTS

The concept and resulting technical effects of the present disclosure will be clearly and completely described below with reference to the embodiments so as to fully understand the objects, features and effects of the present disclosure. Apparently, the described embodiments are merely a part rather than all of the embodiments of the present disclosure. All other embodiments obtained by a person of ordinary skill in the art based on the embodiments of the present disclosure without creative efforts shall fall within the scope of protection of the present disclosure.

Embodiments of the present disclosure are described in detail below, examples of which are illustrated in the accompanying drawings, in which like or similar reference numerals refer to the same or similar elements or elements having the same or similar function throughout. The embodiments described below with reference to the accompanying drawings are exemplary only for explaining the present disclosure and are not to be construed as limiting the present disclosure.

In the description of the present disclosure, it needs to be understood that the orientation or positional relationship indicated by terms "upper", "lower", "front ", "back", "left" and "right" is based on the orientation or positional relationship shown in the drawings only for convenience of description of the present disclosure and simplification of description rather than indicating or implying that the device or element referred to must have a particular orientation, be constructed and operate in a particular orientation, and thus are not to be construed as limiting the present disclosure.

In the description of the present disclosure, the meaning of "several" is more than one, the meaning of "a plurality" is more than two, "greater than", "less than", "more than" and the like are understood to be exclusive of the present number, and "above", "below", "within" and the like are understood to be inclusive of the present number. "First" and "second", if referred to, should be construed as being used to distinguish technical features rather than indicating or implying relative importance or implying a number of the indicated technical features or implying a sequential relationship of the indicated technical features.

In the description of the present disclosure, unless expressly specified otherwise, words such as arrangement, installation and connection are to be understood broadly, and the specific meanings of the words in the invention can be reasonably determined by a person skilled in the art in combination with the specific contents of the technical solution.

In the description of the present disclosure, reference to "one embodiment," "some embodiments," "an illustrative embodiment," "an example," "a specific example," or "some examples," or the like, means that a particular feature, structure, material, or characteristic described in connection with the embodiment or example is included in at least one embodiment or example of the present disclosure. In this specification, schematic expressions for the above terms do not necessarily refer to the same embodiments or examples. Moreover, the particular features, structures, materials, or characteristics described may be combined in a suitable manner in any one or more embodiments or examples.

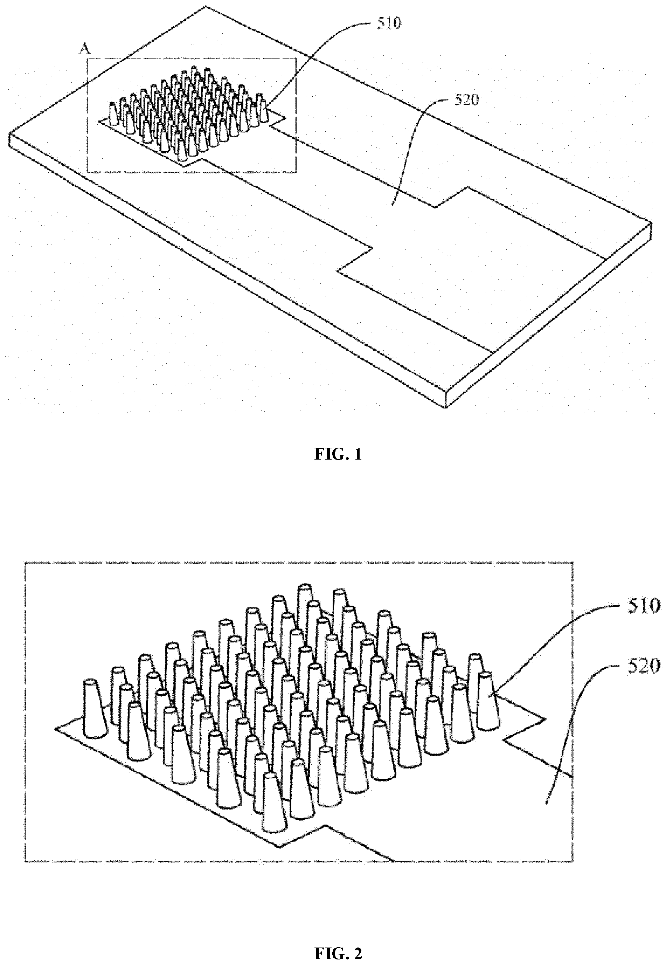

Referring to FIG. 1, a schematic diagram of a structure of a 3D microelectrode is illustrated, and FIG. 2 is a partial enlarged view of a portion A in FIG. 1. The 3D microelectrode in the example is an electrode array, and the 3D microelectrode comprises a substrate portion 520 and a protruded portion 510 fixed to the substrate portion 520, and a single electrode is in a circular truncated cone shape. It may be understood that the shape of the single electrode is not limited to the circular truncated cone shape, and may also be changed into a cone shape, a cylinder shape, a triangular prism shape, a prism shape, a sphere shape and the like according to actual requirements.

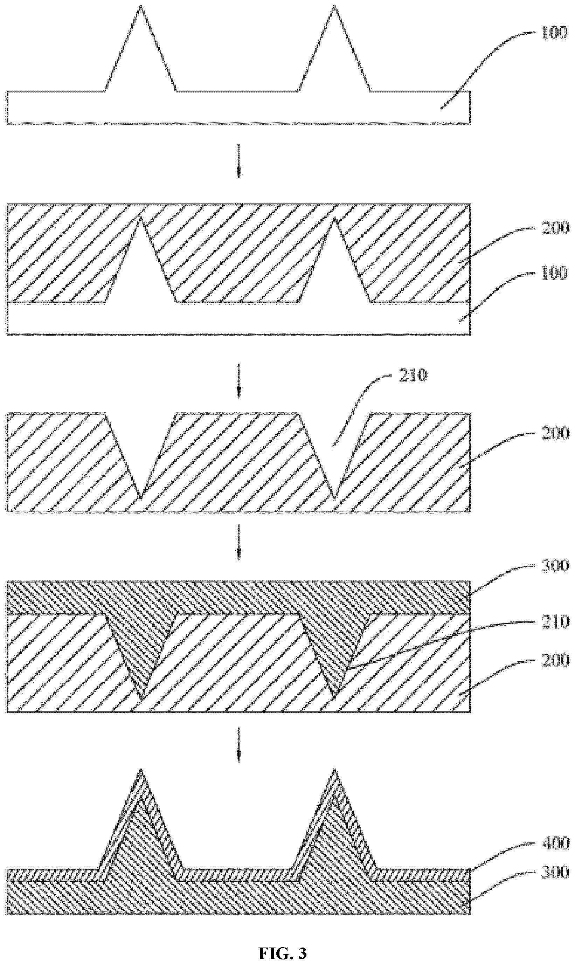

In conjunction with FIG. 3, a manufacturing process of a 3D microelectrode in FIG. 1 is specifically described below:
(1) According to a structure design of a 3D microelectrode in FIG. 1, the 3D model 100 is printed by using a 3D printer, the single conical electrode in the 3D model 100 has a bottom circle radius of 50 µm and has a height of 500 µm, and the distance between the conical electrodes is 150 µm.
(2) A PDMS solution is formed by mixing a PDMS prepolymer and a curing agent in a mass ratio of 10: 1. The printed 3D model 100 is used as a mold, the PDMS solution is poured on the 3D model 100, and then a PDMS flexible mold 200 is formed after demolding. The PDMS flexible mold 200 is provided with a cavity 210, and the cavity 210 can be fitted to the 3D model 100 due to the fact that the PDMS flexible mold 200 is manufactured by demolding with the 3D model 100 as the mold.
(3) One side, provided with the cavity 210, of the PDMS flexible mold 200 is subjected to silanization treatment. In accordance with the embodiment, a perfluorooctyl trichlorosilane reagent is used for performing vacuum deposition on the mold for 20 minutes so as to perform silanization treatment, a layer of polymer film can be formed on the surface of the treated PDMS flexible mold 200, and the layer of polymer film can enable the PDMS poured subsequently to be separated from the PDMS flexible mold 200. The PDMS solution prepared in the step (2) is poured on the surface, provided with the cavity 210, of the PDMS flexible mold 200, and then the PDMS flexible 3D microelectrode substrate 300 is formed after demolding.
(4) A layer of conductive layer 400 is sputtered on the PDMS flexible 3D microelectrode substrate 300 by using a magnetron sputtering technology. In the embodiment, the conductive layer 400 has a thickness of 200 nm, the conductive layer 400 is made of gold, and the manufacturing of the whole 3D microelectrode is completed after cleaning.

In accordance with the embodiment of the present disclosure, by utilizing the characteristic of high printing precision of the 3D printing technology, the 3D model with the same size as the target 3D microelectrode can be accurately printed. Meanwhile, the 3D model with the ultrahigh microcolumn height can be flexibly designed by using the 3D printing technology, and then the 3D microelectrode with the ultrahigh microcolumn height is prepared by combining two-time PDMS mold-reversing. The microcolumn height of the 3D microelectrode is not limited by a process, and the manufacturing of microcolumn electrode array with higher microcolumns is essential for the development of low-cost and high-sensitive microsensors for chemical and biological substances as the larger electrode surface area can be obtained by manufacturing high microcolumn heights and the large electrode surface area is conducive to acquiring larger response current. The 3D microelectrode with ultrahigh microcolumn height can be prepared by using the manufacturing method provided by the embodiment of the present disclosure, has a better application prospect in the field of electrochemical analysis, and can be used for electrochemical analysis of trace substances. As the 3D microelectrode takes a flexible material as a substrate, such as the PDMS flexible 3D microelectrode substrate 300 in FIG. 3, the formed 3D microelectrode is a flexible electrode and can be applied to the field of electrochemical analysis on wearable devices.

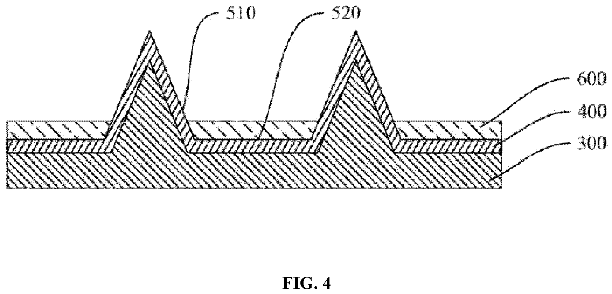

Referring to FIG. 4, to enhance the current density of the electrode and avoid the diffusion overlap of the microcolumn array itself to achieve more efficient electrochemical detection, a non-conductive isolation layer 600 can be manufactured on the substrate portion 520 of the 3D microelectrode while the protruded portion 510 of the 3D microelectrode is exposed. The non-conductive isolation layer 600 can be made of different non-conductive materials, such as silicon nitride, silicon dioxide and non-conductive polymers. Specifically, the manufacturing of the non-conductive isolation layer 600 is achieved by using chemical vapor deposition (PECVD) and lift-off technologies. The manufacturing method specifically comprises the following steps: spin-coating a layer of positive photoresist on the substrate portion 520 of the 3D microelectrode, performing alignment exposure by using a designed mask plate, wherein the area, corresponding to the protruded portion 510 of the 3D microelectrode, of the mask plate is light-proof; and removing the positive photoresist cured on the corresponding substrate portion 520 after developing and baking, and then obtaining a non-conductive isolation layer 600 by plasma enhanced chemical vapor deposition of silicon dioxide. After the uncured positive photoresist on the area corresponding to the protruded portion 510 is stripped by using acetone, the microcolumn electrode array of the flexible substrate with the non-conductive isolation layer 600 can be obtained.

The above description of the embodiments is only intended to help in understanding the method of the present disclosure and its core idea. It should be noted that for those skilled in the art that several improvements and modifications can be made to the present disclosure without departing from the principles of the present disclosure, these improvements and modifications may also fall within the scope of protection of the claims of the present disclosure. Various changes made to these embodiments are apparent for those skilled in the art, and general principles defined herein may be implemented in other embodiments without departing from the spirit or scope of the present disclosure. Hence, the present disclosure is not limited to the embodiments disclosed herein, but is to conform to the widest scope in accordance with principles and novel features disclosed herein.

## Claims

1. A manufacturing method for a 3D microelectrode, comprising the following steps:
(1) manufacturing a 3D model of a 3D microelectrode;
(2) pouring a flexible material into the 3D model, and performing demolding so as to form a flexible mold having a cavity, wherein the cavity of the flexible mold can be fitted to the 3D model;
(3) performing silanization treatment on the flexible mold, then pouring a flexible material into the surface of the flexible mold having the cavity, and performing demolding so as to form a flexible 3D microelectrode substrate; and
(4) manufacturing a conductive layer on the flexible 3D microelectrode substrate so as to form the 3D microelectrode.

2. The manufacturing method for the 3D microelectrode according to claim 1, wherein the flexible material is selected from any one of PDMS, PET and polyimide.

3. The manufacturing method for the 3D microelectrode according to claim 2, wherein the flexible material is a PDMS solution, and a mass ratio of a PDMS prepolymer to a curing agent in the PDMS solution is 10: 1.

4. The manufacturing method for the 3D microelectrode according to claim 1, wherein the 3D microelectrode is an electrode array.

5. The manufacturing method for the 3D microelectrode according to claim 4, wherein a single electrode in the electrode array is a circular truncated-cone-shaped electrode, a conical electrode, a cylindrical electrode, a triangular prism-shaped electrode, a prism-shaped electrode, or a spherical electrode.

6. The manufacturing method for the 3D microelectrode according to claim 5, wherein the circular truncated-cone-shaped electrodes each have a bottom circle radius of 10 µm to 100 µm and a height of 100 µm to 2 mm, and the distance between the circular truncated-cone-shaped electrodes is 100 µm to 500 µm.

7. The manufacturing method for the 3D microelectrode according to claim 4, wherein the column height of a single electrode in the electrode array ranges from 5 µm to 2 mm.

8. The manufacturing method for the 3D microelectrode according to claim 1, wherein the 3D model of the 3D microelectrode is manufactured by using a 3D printing technology.

9. The manufacturing method for the 3D microelectrode according to claim 1, wherein the conductive layer in the step (4) is a conductive metal layer or a conductive polymer layer.

10. The manufacturing method for the 3D microelectrode according to claim 1 or 9, wherein the conductive layer has a thickness of 150 nm to 250 nm.

11. The manufacturing method for the 3D microelectrode according to claim 9, wherein the conductive metal layer is made of gold, platinum or indium tin oxide.

12. The manufacturing method for the 3D microelectrode according to claim 9, wherein in the step (4), the conductive metal layer is manufactured by using a magnetron sputtering process, or the conductive polymer layer is manufactured by coating conductive polymer.

13. The manufacturing method for the 3D microelectrode according to claim 1, wherein the 3D microelectrode is provided with a substrate portion and a protruded portion fixed on the substrate portion, and the method further comprises a step of manufacturing a non-conductive isolation layer on the substrate portion.

14. The manufacturing method for the 3D microelectrode according to claim 13, wherein the non-conductive isolation layer is made of at least one of silicon nitride, silicon dioxide and a non-conductive polymer.

15. The manufacturing method for the 3D microelectrode according to claim 13 or 14, wherein the non-conductive isolation layer is manufactured on the substrate portion by using chemical vapor deposition and lift-off technologies.
